(19) Europäisches Patentamt
European Patent Office
Office européen des brevets

(11) Numéro de publication: **0 052 558**
**B1**

# (12) FASCICULE DE BREVET EUROPEEN

(45) Date de publication du fascicule du brevet: **03.10.84**

(51) Int. Cl.$^3$: **C 07 C 43/225**, C 07 C 43/29, C 07 C 79/35, C 07 C 121/75, C 07 C 103/26, C 07 C 103/30, C 07 C 93/26, C 07 C 41/16

(21) Numéro de dépôt: **81401771.1**

(22) Date de dépôt: **06.11.81**

## (54) Nouveaux bromopolyfluoroalkyl éthers aromatiques et leur procédé de préparation.

(30) Priorité: **13.11.80 FR 8024116**
**25.05.81 FR 8110319**

(43) Date de publication de la demande:
**26.05.82 Bulletin 82/21**

(45) Mention de la délivrance du brevet:
**03.10.84 Bulletin 84/40**

(84) Etats contractants désignés:
**BE CH DE FR GB IT LI NL SE**

(56) Documents cités:
**CHEMICAL ABSTRACTS, vol. 81, no. 17, 28 octobre 1974, page 474 ref. 104876d Columbus, Ohio, US V.E. PLATONOV et al.: "Copyrolysis of pentafluorophenol with tetrafluoro-ethylene and bromine"**
**THE JOURNAL OF ORGANIC CHEMISTRY, vol. 44, no. 16, 3 août 1979 American Chemical Society A.E. FEIRING: "Chemistry in Hydrogen Floride. 7. A Novel Synthesis of Aryl Trifluoromethyl Ethers", pages 2907-2910**
**TETRAHEDRON LETTERS, vol. 22, no. 4, 1981, pages 323-326 Pergamon Press Ltd., GB I. RICO et al.: "Synthèse de Composés Aromatiques comportant les Groupements OCF2Br et SCF2Br" I - Action du *dibromodifluoro-méthyne sur les thiophénate et phénate* de potassium**

(73) Titulaire: **RHONE-POULENC SPECIALITES CHIMIQUES, "Les Miroirs" 18, Avenue d'Alsace, F-92400 Courbevoie (FR)**

(72) Inventeur: **Rico, Isabelle, Villa Laurencia 19, rue de la Butte aux Cailles, F-75013 Paris (FR)**
Inventeur: **Wakselman, Claude, 18, rue du Clos d'Alençon, F-91120 Villebon/sur/Yvette (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 052 558 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention a pour objet de nouveaux bromopolyfluoroalkyléthers aromatiques. L'invention concerne également un procédé de préparation de ces composés.

Les nouveaux composés aromatiques selon la présente invention ont pour formule générale:

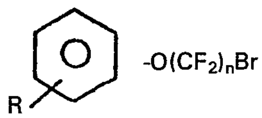

(I)

dans laquelle:

— n est égal à 1 ou 2

— et R représente au moins un substituant choisi parmi le groupe comprenant l'hydrogène, les radicaux alkyle ayant de 1 à 12 atomes de carbone, le radical phényle, les radicaux alkoxy ayant de 1 à 12 atomes de carbone, le radical phényloxy et les radicaux $NO_2$, CN, F, Cl, Br, I, $CF_3$, $CONR_1R_2$ et $NR_1R_2$ où $R_1$ et $R_2$ identiques ou différents représentent chacun un hydrogène ou un radical alkyle ayant de 1 à 6 atomes de carbone, le noyau benzénique n'étant jamais perfluoré.

Les composés de formule I ont un grand intérêt industriel dans la mesure où ils peuvent être utilisés en tant qu'intermédiaires de synthèse pour la préparation de produits ayant une activité phytosanitaire ou pharmaceutique. C'est ainsi en particulier que par échange d'halogènes, ces composés donnent les perfluoroalkyléthers correspondants qui sont des intermédiaires bien connus pour la synthèse des produits précités.

L'invention concerne également un procédé pour la préparation des composés de formule I caractérisé en ce que l'on fait réagir un composé de formule:

$$Br(CF_2)_n\text{-}X \qquad (II)$$

où X représente le chlore ou le brome et où n est égal à 1 ou 2 avec un phénate de formule:

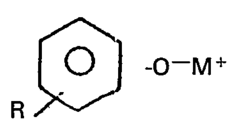

(III)

où R à la signification précédente et $M^+$ représente un cation dérivé d'un métal alcalin, dans un solvant aprotique polaire anhydre en présence d'un thiol agissant comme initiateur de la réaction.

Selon un mode de réalisation préférentiel de l'invention, on utilise comme composé II $Br(CF_2)_n Br$ qui permet d'atteindre les meilleurs rendements.

La demanderesse a également constaté que pour atteindre les meilleurs rendements, il est préférable d'utiliser un phénate de potassium. En effet, c'est lorsque ce dernier est utilisé que la quantité de produit secondaire (produit correspondant ayant le groupement-$O(CF_2)_nH$) formée est la plus faible.

Le solvant aprotique polaire mis en oeuvre est de préférence un solvant dont la constante diélectrique est supérieure à 15. On peut citer comme exemples de solvants convenant particulièrement bien à la mise en oeuvre du procédé de l'invention, le diméthylformamide, le diméthylacétamide, le diméthylsulfoxyde, l'hexaméthylènephosphorotriamide, la N-méthylpyrrolidone et le sulfolane.

Le solvant utilisé doit être aprotique pour éviter la formation du produit secondaire ayant le groupement $O(CF_2)_nH$ et polaire pour exalter la réactivité du phénate.

Selon un mode de réalisation tout particulièrement préféré, on utilise comme solvant le diméthylformamide.

Selon une caractéristique fondamentale du procédé selon l'invention, il est nécessaire pour initier la réaction, d'ajouter au milieu réactionnel un thiol. Ce thiol que l'on peut représenter par la formule $R_3SH$ libère, puisque l'on se trouve en milieu basique, l'ion $R_3S^-$ dont le caractère nucléophile permet l'initiation. $R_3$ peut représenter tout radical organique hydrocarboné (alkyle ou aryle) ou contenant des hétéroatomes.

On peut citer comme exemples de thiols utilisables: les alcanethiols, comme l'éthanethiol, le propanethiol, le butanethiol, les thiophénols et les benzylmercaptans.

On préfère utiliser le propanethiol car les produits secondaires dont il est responsable sont suffisamment volatils pour être éliminés facilement.

Le thiol est utilisé en quantité telle que le rapport molaire du thiol au phénate est compris entre environ 0,05 et environ 0,2. Encore plus préférentiellement, ce rapport est compris entre 0,07 et 0,15.

On met en oeuvre de préférence les composés II et III en quantité telle que le rapport molaire du composé II au phénate III est compris entre environ 1 et environ 5. Encore plus préférentiellement, ce rapport est compris entre environ 1 et environ 3.

Le solvant est utilisé en quantité tel que le nombre de mole du phénate par litre de solvant est compris entre environ 0,1 et environ 0,5 et encore plus préférentiellement entre 0,15 et 0,3.

On effectue généralement la réaction à une température comprise entre environ 20 et 100°C et plus particulièrement entre 20 et 60°C.

On opère de préférence sous pression atmosphérique bien que des pressions supérieures ou inférieures à la pression atmosphérique ne soient pas exclues du domaine de l'invention.

Les temps de réactionn nécessaires à l'accomplissement de la réaction selon l'invention peuvent varier dans de larges limites. Ils sont généralement compris entre 3 et 12 heures.

La demanderesse a constaté que pour mettre en oeuvre le procédé selon l'invention dans de meilleures conditions, il était souhaitable d'utiliser un solvant bien dégazé c'est-à-dire débarrassé de l'oxygène de l'air et de maintenir le milieu réactionnel sous agitation.

De plus, pour une bonne mise en oeuvre de l'invention, il convient d'utiliser un phénate exempt d'eau. De ce fait, il est recommandé de sécher le phénate dans une étage préalable.

En effet, la présence d'eau dans le milieu réactionnel favorise la formation du produit secondaire correspondant comportant le groupement $O(CF_2)_nH$.

Les composés de formule II et III sont préparés selon des techniques bien connues de l'homme de l'art.

L'invention va être maintenant plus complètement décrite à l'aide des exemples qui vont suivre. Ceux-ci ne sauraient être interprétés comme limitant de façon quelconque l'invention.

*Exemple 1*

Préparation du bromodifluorométhoxy,méthyl-4 benzène

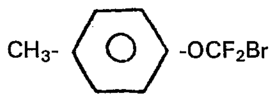 par action de $CF_2Br_2$ sur

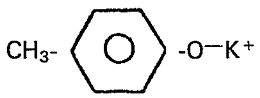

Dans un ballon surmonté d'un réfrigérant à carboglace et contenant 400 ml de diméthylformamide anhydre dégazé à l'argon, on dissout 14,6 g (0,1 mole) de:

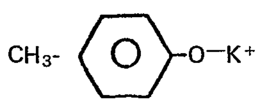

et 0,76 g (0,01 mole) de propanethiol à 20°C. On ajoute ensuite goutte à goutte sous agitation 42 g (0,2 mole) de $CF_2Br_2$. La température augmente de 20 à 30°C. On laisse le mélange sous agitation pendant quatre heures à température ambiante. Le solvant et KBr qui se forme au cours de la réaction sont éliminés par addition de 400 ml d'eau acidifiée par HCl (à 17%). L'huile qui décante est récupérée. Après purification par un entraînement à la vapeur d'eau, cette huile est séchée sur carbonate de sodium. On sépare par distillation à la bande tournante 5 g d'un produit dont le point d'ébullition est $Eb_{34\,mmHg}$ = 90°C et dont l'analyse RMN du proton et du fluor révèlent la formule:

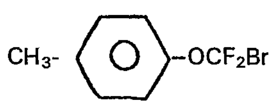

*Exemple 2*

Préparation du bromodifluorométhoxy,méthyl-4 benzène

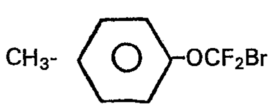 par action de $CF_2BrCl$ sur

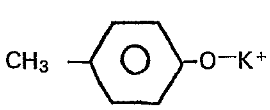

Dans un ballon surmonté d'un réfrigérant à carboglace contenant 400 ml de diméthylformamide anhydre dégazé à l'argon, on dissout 14,6 g (0,1 mole) de:

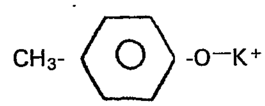

et 0,76 g (0,01 mole) de propanethiol à 20°C. On ajoute ensuite d'un seul coup 33 g (0,2 mole) de $CF_2BrCl$ préalablement condensés dans un bain carboglace-acétone. La température s'élève de quelques degrés. On opère ensuite comme dans l'exemple 1. On sépare finalement par distillation à la bande tournante 2,8 g de:

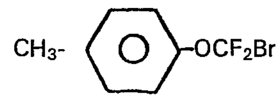

*Exemple 3*

Préparation du bromodifluorométhoxybenzène

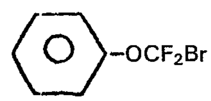

par action de $CF_2Br_2$ sur 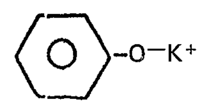

On opère comme dans l'exemple 1 avec 13,2 g (0,1 mole) de:

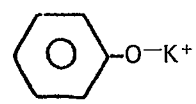

Par distillation à la bande tournante on sépare 2 g d'un produit dont le point d'ébullition est $Eb_{34\,mmHg}$ = 70°C et dont l'analyse RMN du proton et du fluor révèlent la formule:

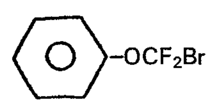

*Exemple 4*

Préparation du bromodifluorométhoxy,chloro-4 benzène

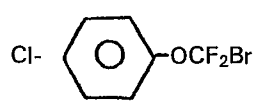 par action de $CF_2Br_2$ sur

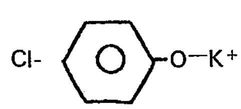

On opère comme dans l'exemple 1 avec 16,6 g (0,1 mole) de:

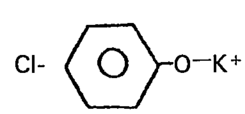

Par distillation à la bande tournante on sépare 4,1 g d'un produit dont le point d'ébullition est $Eb_{32\ mmHg}$ = 94°C et dont l'analyse RMN du proton et du fluor révèlent la formule:

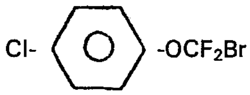

*Exemple 5*

Préparation du bromodifluorométhoxy,nitro-4 benzène

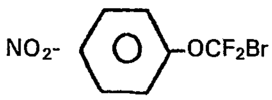 par action de $CF_2Br_2$ sur

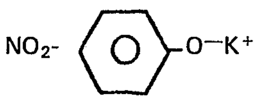

On opère comme dans l'exemple 1 avec 17,7 g (0,1 mole) de:

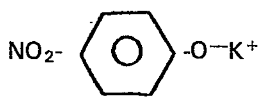

Par chromatographie sur plaque de silice (Eluat: benzène) on sépare 6,7 g d'un produit dont le point de fusion est P.F. = 48°C et dont l'analyse RMN du proton et du fluor révèlent la formule:

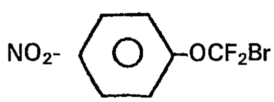

*Exemple 6*

Préparation du β-bromotétrafluoroéthoxyméthyl-4 benzène

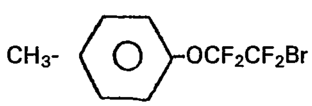

par action de

$BrCF_2CF_2Br$ sur 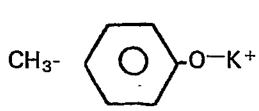

Dans un ballon surmonté d'un réfrigérant à carboglace et contenant 400 ml de diméthylformamide anhydre dégazé à l'argon, on dissout 14,6 g (0,1 mole) de:

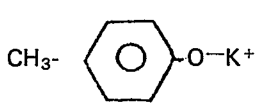

et 0,76 g (0,01 mole) de propanethiol à 20°C. On ajoute ensuite goutte à goutte sous agitation 39 g (0,15 mole) de $BrCF_2CF_2Br$. La température augmente de 20 à 24°C. On laisse le mélange sous agitation pendant 1 h à 40°C, puis 12 h à température ambiante. Le solvant et KBr qui se forme au cours de la réaction sont éliminés par addition de 400 ml d'eau acidifiée par HCl (à 17%). L'huile qui décante est récupérée. Après purification par un entraînement à la vapeur d'eau, cette huile est séchée sur carbonate de sodium. On sépare par distillation à la bande tournante 10,9 g d'un produit dont le point d'ébullition est $Eb_{30\ mmHg}$ = 86°C et dont l'analyse RMN du proton et du fluor révèlent la formule:

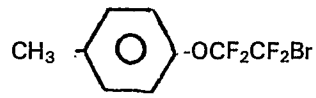

*Exemple 7*

Préparation du β-bromotétrafluoroéthoxybenzène

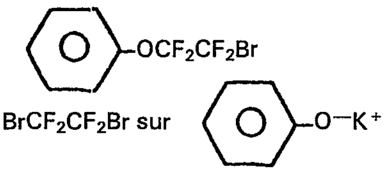

par action de $BrCF_2CF_2Br$ sur

On opère comme dans l'exemple 6 avec 13,2 g (0,1 mole) de

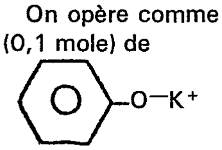

Par distillation à la bande tournante, on sépare 7 g d'un produit dont le point d'ébullition est $Eb_{40\ mmHg}$ = 76°C et dont l'analyse RMN du proton et du fluor révèlent la formule:

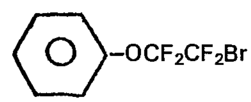

*Exemple 8*

Préparation du β-bromotétrafluoroéthoxy,chloro-4 benzène

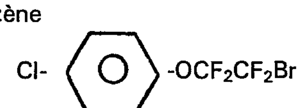

par action de $BrCF_2CF_2Br$ sur 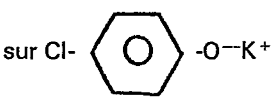

On opère comme dans l'exemple 6 avec 16,6 g (0,1 mole) de

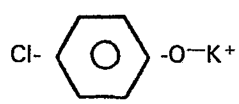

Par distillation à la bande tournante, on sépare 5,8 g d'un produit dont le point d'ébullition est $Eb_{30\ mmHg}$ = 80°C et dont l'analyse du proton et du fluor révèlent la formule:

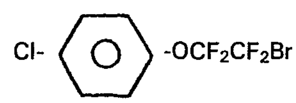

*Exemple 9*

Préparation du β-bromotétrafluoroéthoxy,nitro-4 benzène

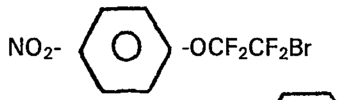

par action de $BrCF_2CF_2Br$ sur 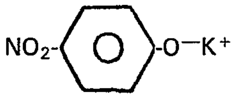

On opère comme dans l'exemple 6 avec 17,7 g (0,1 mole) de

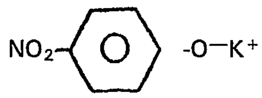

Par chromatographie sur plaque de silice (Eluat: benzène), on sépare 2 g d'un produit dont le point de fusion est P.F. = 65°C et dont l'analyse RMN du proton et du fluor révèlent la formule:

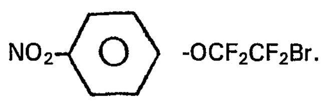

**Revendications**

1. Bromopolyfluoroalkyléthers aromatiques de formule:

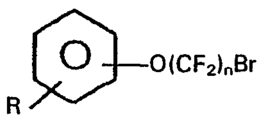 (I)

dans laquelle n est égal à 1 ou 2, R représente au moins un substituant choisi parmi le groupe comprenant l'hydrogène, les radicaux alkyle ayant de 1 à 12 atomes de carbone, le radical phényle, les radicaux alkoxy ayant de 1 à 12 atomes de carbone, le radical phényloxy et les radicaux $NO_2$, N, Cl, F, Br, I, $CF_3$, $CONR_1R_2$ et $NR_1R_2$ où $R_1$ et $R_2$ identiques ou différents représentent chacun un hydrogène ou un radical alkyle ayant de 1 à 6 atomes de carbone, le noyau benzénique n'étant jamais perfluoré.

2. Bromopolyfluoroalkyléther aromatique selon la revendication 1, de formule:

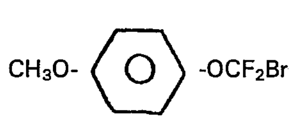

3. Bromopolyfluoroalkyléther aromatique selon la revendication 1, de formule:

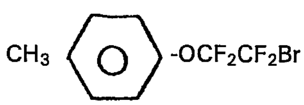

4. Bromopolyfluoroalkyléthers aromatiques selon la revendication 1, de formule:

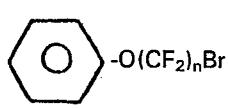

5. Bromopolyfluoroalkyléthers aromatiques selon la revendication 1, de formule:

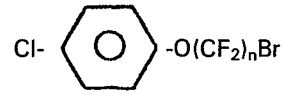

6. Bromopolyfluoroalkyléthers aromatiques selon la revendication 1, de formule:

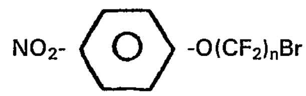

7. Procédé pour la préparation de composés selon l'une quelconque des revendications précédentes, caractérisé en ce que l'on fait réagir un composé de formule:

$$Br(CF_2)_nX \qquad (II)$$

où X représente le chlore ou le brome et n est tel que défini dans la revendication 1 avec un phénate de formule:

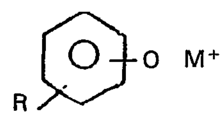 (III)

où R est tel que défini dans la revendication 1 et $M^+$ représente un cation dérivé d'un metal alcalin, dans un solvant aprotique polaire anhydre en présence d'un thiol agissant comme initiateur de la réaction.

8. Procédé selon la revendication 7, caractérisé en ce que dans la formule II X = Br.

9. Procédé selon la revendication 7, caractérisé en ce que dans la formule III $M^+$ représente le cation dérivé du potassium.

10. Procédé selon la revendication 7, caractérisé en ce que le solvant aprotique polaire est choisi parmi le groupe comprenant le diméthylformamide, le diméthylsulfoxyde, l'hexaméthylènephosphorotriamide, la N-méthylpyrrolidone et le sulfolane.

11. Procédé selon la revendication 10, caractérisé en ce que le solvant est le diméthylformamide.

12. Procédé selon la revendication 7, caractérisé en ce que le thiol est choisi parmi le groupe comprenant les alcanethiols, les thiophénols et les benzylmercaptans.

13. Procédé selon la revendication 12, caractérisé en ce que le thiol est le propanethiol.

14. Procédé selon la revendication 7, caractérisé en ce que le rapport molaire du thiol au phénate est compris entre 0,05 et 0,2.

15. Procédé selon la revendication 7, caractérisé en ce que le rapport molaire du composé II au composé III est compris entre 1 et 5.

16. Procédé selon la revendication 7, caractérisé en ce que le nombre de mole de phénate par litre de solvant est compris entre 0,1 et 0,5.

17. Procédé selon l'une quelconque des revendications précédentes, caractérisé en ce qu'on opère à une température comprise entre 20 et 100°C sous pression atmosphérique.

**Patentansprüche**

1. Aromatische Brompolyfluoralkylether der Formel

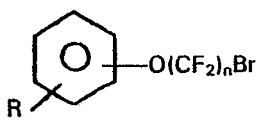 (I)

in der n = 1 oder 2 ist, R mindestens ein Substituent ist aus der Gruppe von Wasserstoff, den Alkylresten mit 1 bis 12 Kohlenstoffatomen, dem Phenylrest, den Alkoxyresten mit 1 bis 12 Kohlenstoffatomen, dem Phenyloxyrest und den Resten $-NO_2$, -CN, -Cl, -F, -Br, -I, $-CF_3$, $-CONR_1R_2$ und $-NR_1R_2$ mit identischen oder verschiedenen $R_1$ und $R_2$, die jeweils ein Wasserstoffatom oder einen Alkylrest mit 1 bis 6 Kohlenstoffatomen bedeuten, wobei der Benzolkern nie perfluoriert ist.

2. Aromatischer Brompolyfluoralkylether nach Anspruch 1 mit der Formel

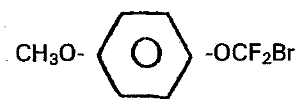

3. Aromatischer Brompolyfluoralkylether nach Anspruch 1 mit der Formel

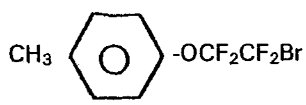

4. Aromatischer Brompolyfluoralkylether nach Anspruch 1 mit der Formel

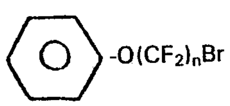

5. Aromatischer Brompolyfluoralkylether nach Anspruch 1 mit der Formel

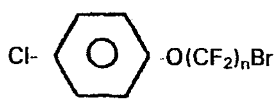

6. Aromatischer Brompolyfluoralkylether nach Anspruch 1 mit der Formel

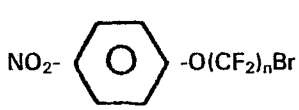

7. Verfahren zur Herstellung von Verbindungen nach einem oder mehreren der vorangehenden Ansprüche, dadurch gekennzeichnet, dass man eine Verbindung der Formel

$$Br(CF_2)_nX \quad (II)$$

in der X Chlor oder Brom darstellt und n so wie in Anspruch 1 definiert ist, mit einem Phenolat der Formel

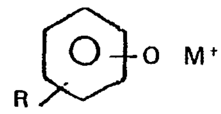 (III)

in der R so wie in Anspruch 1 definiert ist und $M^+$ ein Alkalimetallkation darstellt, in einem aprotischen, polaren, wasserfreien Lösungsmittel in Anwesenheit eines Thiols als Reaktionsinitiator umsetzt.

8. Verfahren nach Anspruch 7, dadurch gekennzeichnet, dass in der Formel II X = Br ist.

9. Verfahren nach Anspruch 7, dadurch gekennzeichnet, dass in der Formel III $M^+$ das Kaliumkation ist.

10. Verfahren nach Anspruch 7, dadurch gekennzeichnet, dass das aprotische polare Lösungsmittel aus der Gruppe von Dimethylformamid, Dimethylsulfoxid, Hexamethylenphosphorsäuretriamid, N-Methylpyrrolidon und Sulfolan gewählt wird.

11. Verfahren nach Anspruch 10, dadurch gekennzeichnet, dass das Lösungsmittel Dimethylformamid ist.

12. Verfahren nach Anspruch 7, dadurch gekennzeichnet, dass das Thiol aus der Gruppe der Alkanthiole, Thiophenole und Benzylmercaptane gewählt wird.

13. Verfahren nach Anspruch 12, dadurch gekennzeichnet, dass das Thiol Propanthiol ist.

14. Verfahren nach Anspruch 7, dadurch gekennzeichnet, dass das Molverhältnis zwischen Thiol und Phenolat zwischen 0,05 und 0,2 liegt.

15. Verfahren nach Anspruch 7, dadurch gekennzeichnet, dass das Molverhältnis zwischen Verbindung II und Verbindung III 1 bis 5 beträgt.

16. Verfahren nach Anspruch 7, dadurch gekennzeichnet, dass die Molzahl an Phenolat pro Liter Lösungsmittel zwischen 0,1 und 0,5 liegt.

17. Verfahren nach einem oder mehreren der vorangehenden Ansprüche, dadurch gekennzeichnet, dass man bei einer Temperatur zwischen 20 und 100°C und unter Atmosphärendruck arbeitet.

**Claims**

1. Aromatic bromopolyfluoroalkyl ethers of the formula:

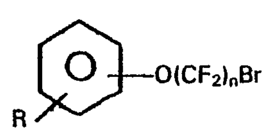 (I)

in which n is 1 or 2, R represents at least one substituent chosen from among the group comprising hydrogen, alkyl radicals having from 1 to 12 carbon atoms, the phenyl radical, alkoxy radicals having from 1 to 12 carbon atoms, the phenoxy radical and the radicals $NO_2$, N, Cl, F, Br, I, $CF_3$, $CONR_1R_2$ and $NR_1R_2$, where $R_1$ and $R_2$, which may be identical or different, each represent a hydrogen or an alkyl radical having from 1 to 6 carbon atoms, and in which the benzene nucleus is never perfluorinated.

2. An aromatic bromopolyfluoroalkyl ether according to Claim 1, of the formula:

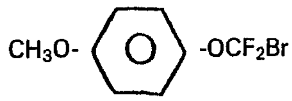

3. An aromatic bromopolyfluoroalkyl ether according to Claim 1, of the formula:

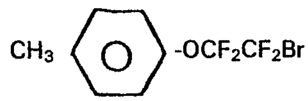

4. An aromatic bromopolyfluoroalkyl ether according to Claim 1, of the formula:

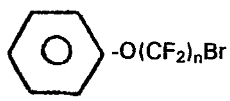

5. An aromatic bromopolyfluoroalkyl ether according to Claim 1, of the formula:

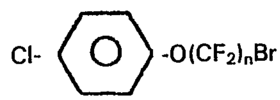

6. An aromatic bromopolyfluoroalkyl ether according to Claim 1, of the formula:

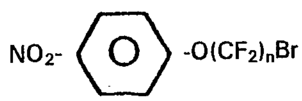

7. Process for the preparation of compounds according to any one of the preceding claims, characterised in that a compound of the formula:

$Br(CF_2)_nX$ (II)

where X represents chlorine or bromine and n is as defined in Claim 1, is reacted with a phenate of the formula:

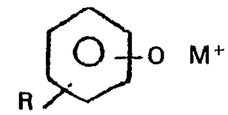

(III)

where R is a defined in Claim 1 and $M^+$ represents a cation derived from an alkali metal, in an anhydrous polar aprotic solvent in the presence of a thiol acting as a reaction initiator.

8. Process according to Claim 7, characterised in that in formula II X = Br.

9. Process according to Claim 7, characterised in that in formula III $M^+$ represents the cation derived from potassium.

10. Process according to Claim 7, characterised in that the polar aprotic solvent is chosen from among the group comprising dimethylformamide, dimethylsulphoxide, hexamethylenephosphorotriamide, N-methylpyrrolidone and sulpholane.

11. Process according to Claim 10,, characterised in that the solvent is dimethylformamide.

12. Process according to Claim 7, characterised in that the thiol is chosen from among the group comprising the alkanethiols, the thiophenols and the benzylmercaptans.

13. Process according to Claim 12, characterised in that the thiol is propanethiol.

14. Process according to Claim 7, characterised in that the molar ratio of the thiol to the phenate is between 0.05 and 0.2.

15. Process according to Claim 7, characterised in that the molar ratio of the compound II to the compound III is between 1 and 5.

16. Process according to Claim 7, characterised in that the number of moles of phenate per litre of solvent is between 0.1 and 0.5.

17. Process according to any one of the preceding claims, characterised in that it is carried out at a temperature of between 20 and 100°C under atmospheric pressure.